# EUROPÄISCHE PATENTSCHRIFT

(11) **EP 2 946 749 B1**
(45) Veröffentlichungstag und Bekanntmachung des Hinweises auf die Patenterteilung: **20.06.2018**
(21) Anmeldenummer: 15001546.9
(22) Anmeldetag: 20.05.2015
(51) Int. Cl.: A61C 19/00, A61C 1/00, A61B 90/70

(54) **VORRICHTUNG ZUR INNENDURCHSPÜLUNG VON DENTALEN HANDSTÜCKEN**
DEVICE FOR INTERNAL WASHING OF DENTAL HANDPIECES
DISPOSITIF DE RINÇAGE INTERNE DE PIÈCES À MAIN DENTAIRES

(30) Priorität: 21.05.2014 DE 102014107136
(43) Veröffentlichungstag der Anmeldung: 25.11.2015
(73) Patentinhaber: Miele & Cie. KG, 33332 Gütersloh (DE)
(72) Erfinder: Penner, Roman, 33613 Bielefeld (DE)

(56) Entgegenhaltungen:
- CN-A- 101 411 882
- DE-U1- 29 624 425

## Beschreibung

Die Erfindung betrifft eine Vorrichtung zur Innendurchspülung von dentalem Spülgut, insbesondere von Turbinen und/oder Hand- und Winkelstücken, mit einer Kupplungseinrichtung zum Anschluss des Spülguts an einen mit einer Anschlusseinrichtung einer Rohrleitung verbundenen oder damit verbindbaren Anschlussstutzen.

Eine Spülvorrichtung der eingangs genannten Art ist aus der DE 296 24 425 U1 bekannt. Die hier beschriebene Spülvorrichtung dient dem Anschluss eines zu reinigenden Spülguts an eine von einem Instrumenteneinsatz bereitgestellte Rohrleitung. Im bestimmungsgemäßen Verwendungsfall gelangt über diese Rohrleitung Spülflotte zur Spülvorrichtung, über die dann eine Innenbeaufschlagung des zu reinigenden Spülguts mit Spülflotte erfolgt.

Die aus der DE 296 24 425 U1, und ebenfalls aus der CN 101411882 A, vorbekannte Spülvorrichtung verfügt über ein rohrseitiges Anschlussteil einerseits und ein dieses im bestimmungsgemäßen Verwendungsfall verschließendes, kappenähnliches Oberteil andererseits. Innerhalb des von Anschlussteil und Oberteil ausgebildeten Volumenraums ist ein Kupplungsteil aus einem elastischen Material angeordnet. Dieses Kupplungsteil dient der flüssigkeitsdichten Spülgutaufnahme und verfügt über eine Einstecköffnung, durch die hindurch im bestimmungsgemäßen Verwendungsfall das zu reinigende Spülgut geführt ist.

Im bestimmungsgemäßen Verwendungsfall ist das zu reinigende Spülgut in Entsprechung seiner geometrischen Außenabmessungen mit einem hinsichtlich seiner Einstecköffnung hierzu passend ausgebildeten Kupplungsteil zu bestücken. Das zu reinigende Spülgut ist alsdann mit seinem das Kupplungsteil tragenden Abschnitt in das Anschlussteil einzusetzen, und das Anschlussteil ist zur Lagesicherung des Kupplungsteils mit dem Oberteil zu verschließen, zu welchem Zweck bevorzugterweise zwischen Anschlussteil und Oberteil eine Schraubverbindung ausgebildet ist.

Obgleich sich die vorbeschriebene Spülvorrichtung im alltäglichen Praxiseinsatz bewährt hat, ist diese nicht frei von Nachteilen. So ist es für eine flüssigkeitsdichte Aufnahme des Spülguts erforderlich, je nach geometrischen Ausgestaltung des Spülguts ein Kupplungsteil mit einer entsprechend ausgebildeten Spülgut-Einstecköffnung zu verwenden. Es müssen deshalb in der Ausbildung der Einstecköffnung unterschiedlich ausgestaltete Kupplungsteile bevorratet und je nach zu reinigendem Spülgut manuell ausgewählt und auf das zu reinigende Spülgut aufgesetzt werden. Dies ist in nachteiliger Weise nicht nur aufwändig in der Handhabung, es besteht vor allem die Gefahr einer nicht ordnungsgemäßen Innenreinigung des Spülguts bei Fehlbestückung. Insoweit stellen unterschiedliche Anschlussgeometrien zu reinigender Spülgüter ein erhebliches Handhabungsproblem dar.

Hinzukommt, dass das außenseitig abdichtend am Spülgut anliegende Kupplungsteil hinsichtlich einer Außenreinigung einen Spülschatten darstellt. Im Kontaktbereich zwischen Spülgut einerseits und Kupplungsteil andererseits kann eine Außenreinigung nicht stattfinden, was insbesondere insoweit bedenklich ist, als dass dieser Bereich häufig durch den Griffbereich des zu reinigenden Spülguts gebildet ist. Gerade dieser Bereich bedarf deshalb einer Außenreinigung, was aber aufgrund des Anliegens des Kupplungsteils am Spülgut nicht oder nur unzureichend gewährleistet ist. Auch insofern besteht deshalb Verbesserungsbedarf.

Ausgehend vom Vorbeschriebenen ist es die **Aufgabe** der Erfindung, eine Vorrichtung der eingangs genannten Art dahingehend weiterzuentwickeln, die es ermöglicht, bei gleichzeitig vereinfachter Handhabung ein verbessertes Reinigungsergebnis zu erzielen.

Zur **Lösung** dieser Aufgabe wird mit der Erfindung eine Vorrichtung mit den Merkmalen von Anspruch 1 vorgeschlagen. Die erfindungsgemäße Spülvorrichtung verfügt über eine Kupplungseinrichtung einerseits und eine Klemmeinrichtung andererseits. Dabei dient die Kupplungseinrichtung dazu, das zu reinigende Spülgut an einen mit einer Anschlusseinrichtung einer Rohrleitung verbundenen oder damit verbindbaren Anschlussstutzen anzuschließen. Mittels der Kupplungseinrichtung wird demnach der strömungstechnische Anschluss des zu reinigenden Spülguts an einen mit einer Anschlusseinrichtung einer Rohrleitung verbundenen oder damit verbindbaren Anschlussstutzen ausgebildet.

Die Klemmeinrichtung dient indes dazu, das an die Kupplungseinrichtung angeschlossene Spülgut in seiner Lage zu fixieren, wobei erfindungsgemäß vorgesehen ist, dass die Kupplungseinrichtung zur Betätigung der Klemmeinrichtung relativ verfahrbar zum Anschlussstutzen ausgebildet ist. Die Betätigung der Klemmeinrichtung findet demnach mittels der Kupplungseinrichtung statt. Zu diesem Zweck ist konstruktiv vorgesehen, dass die Kupplungseinrichtung relativ verfahrbar zum Anschlussstutzen ausgebildet ist. Infolge einer Verfahrbewegung der Kupplungseinrichtung relativ zum Anschlussstutzen erfolgt mithin eine Betätigung der Klemmeinrichtung.

Die erfindungsgemäße Ausgestaltung erlaubt in vorteilhafterweise eine im Unterschied zum Stand der Technik vereinfachte Handhabung. So wird mit der erfindungsgemäßen Konstruktion erreicht, dass es bei einer bestimmungsgemäßen Bestückung der Kupplungseinrichtung mit einem zu reinigendem Spülgut zu einer Verfahrbewegung derselben kommt, infolgedessen es zur Betätigung der Klemmeinrichtung kommt, was zur Lagefixierung des zu reinigenden Spülguts führt. Ein Bestücken der Kupplungseinrichtung mit einem zu reinigenden Spülgut führt mithin automatisch auch zur Lagefixierung desselben.

Die Lagefixierung des zu reinigenden Spülguts mittels der Klemmeinrichtung erbringt zudem den Vorteil, dass die Spülvorrichtung universell, d. h. unabhängig von der Anschlussgeometrie zu reinigender Spülgüter eingesetzt werden kann. Da im Unterschied zum Stand der Technik anstelle eines formschlüssig mit dem zu reinigenden Spülgut in Verbindung stehenden Kupplungsteils eine kraftschlüssig wirkende Klemmeinrichtung zum Einsatz kommt, erlaubt die erfindungsgemäße Spülvorrichtung eine bestimmungsgemäße Aufnahme von in ihren geometrischen Abmessungen unterschiedlich ausgebildeten Spülgütern. In diesem Zusammenhang erweist sich zudem von Vorteil, dass die Klemmeinrichtung nur über einen vergleichsweise kleinen Kontaktbereich mit dem lagezufixierenden Spülgut in Verbindung steht, womit im bestimmungsgemäßen Anwendungsfall eine im Unterschied zum Stand der Technik sehr viel kleinerer Spülschatten entsteht. Damit erlaubt die erfindungsgemäße Ausgestaltung eine verbesserte Außenreinigung.

Da mit der erfindungsgemäßen Ausgestaltung auf ein formschlüssig am zu reinigenden Spülgut anliegendes Kupplungsteil vollends verzichtet ist, entfällt zudem der dem Stand der Technik anhaftende Nachteil, unterschiedliche Kupplungsstücke für unterschiedlich ausgestaltete Spülgüter vorzuhalten. Zudem ist die Gefahr einer nur unzureichenden Innenreinigung infolge einer Kupplungsteilfehlbestückung überwunden. Damit erbringt die erfindungsgemäße Ausgestaltung nicht nur eine vereinfachte Handhabung, es wird auch eine im Unterschied zum Stand der Technik verbessertes und weniger fehleranfälliges Reinigungsergebnis erzielt.

Die Kupplungseinrichtung ist gemäß einem weiteren Merkmal der Erfindung in Höhenrichtung des Anschlussstutzens relativ verfahrbar zum Anschlussstutzen ausgebildet. Demnach findet eine Bestückung der Kupplungseinrichtung durch das zu reinigende Spülgut in Höhenrichtung des Anschlussstutzens statt, d. h. das zu reinigende Spülgut wird in Höhenrichtung des Anschlussstutzens auf die Kupplungseinrichtung aufgesetzt, wobei infolge eines solchen Aufsetzens eine Verfahrbewegung der Kupplungseinrichtung relativ zum Anschlussstutzen stattfindet, was in der schon vorbeschriebenen Weise zur Betätigung der Klemmeinrichtung führt. Eine solche Verfahrmöglichkeit der Kupplungseinrichtung vereinfacht die Handhabung der erfindungsgemäßen Spülvorrichtung, weil so einerseits Bestückungsrichtung und andererseits Verschieberichtung in Anschlussstutzen-Höhenrichtung verlaufen.

Gemäß einem weiteren Merkmal der Erfindung ist vorgesehen, dass die Klemmeinrichtung eine Bügelfeder aufweist, deren Federschenkel einendseitig jeweils eine mit dem Spülgut zusammenwirkende Klemmbacke aufweisen. Diese Bügelfeder besteht bevorzugterweise aus einer Federbasis sowie zwei Federschenkeln, wobei die Federbasis einerseits in den einen Federschenkel und andererseits in den anderen Federschenkel übergeht. Der Federbasis gegenüberliegend trägt jeder Federschenkel endseitig jeweils eine Klemmbacke. Bei einer bestimmungsgemäßen Lagefixierung eines zu reinigenden Spülgutes liegen diese Klemmbacken am zu reinigenden Spülgut an und klemmen dieses zwischen sich ein, wodurch die Lagefixierung erreicht ist. Diese Stellung der Federschenkel kann auch als Klemmstellung bezeichnet werden. In einer Nicht-Klemmstellung sind die Federschenkel in ihrer Ausgangsposition nach außen verfahren, so dass ein anliegender Klemmkontakt der Klemmbacken am zu reinigenden Spülgut nicht vorliegt.

Die Kupplungseinrichtung stützt sich gemäß einem weiteren Merkmal der Erfindung gegenüber dem Anschlussstutzen oder gegenüber einem mit dem Anschlussstutzen verbundenen Halteelement unter Zwischenschaltung einer Druckfeder ab. Die Druckfeder sorgt mithin dafür, dass sich die Kupplungseinrichtung stets in ihrer Ausgangsposition befindet, in der sie für ein zu reinigendes Spülgut aufnahmebereit ist. Sobald die Kupplungseinrichtung mit einem zu reinigenden Spülgut bestückt und zwecks Betätigung der Klemmeinrichtung verfahren ist, steht die Druckfeder unter Spannung und ist bestrebt, die Kupplungseinrichtung zurück in ihre Ausgangsposition zu verfahren. In der lagefixierten Stellung des Spülguts sorgt eine vorzugsweise von der Klemmeinrichtung bereitgestellten Rast- oder Arretiereinrichtung dafür, dass die Kupplungseinrichtung nicht ungewollt in ihrer Ausgangsposition zurückverfährt.

Die Freigabe der Rast- oder Arretiereinrichtung kann durch manuelle Betätigung, insbesondere durch ein leichtes Einfedern der Federschenkel, erfolgen. Die Druckfeder bewirkt dann eine Zurücküberführung der Kupplungseinrichtung in die Ausgangsstellung, in welcher die Lagefixierung des zu reinigenden Spülguts gelöst ist, so dass eine Entnahme eines gereinigten Spülguts erfolgen kann. Die Rast- oder Arretiereinrichtung ist jedoch vorzugsweise insbesondere so ausgebildet, dass eine Entnahme des an die Kupplungseinrichtung angeschlossenen Spülguts, insbesondere eine Entnahme nach oben, eine Freigabe der Rast- oder Arretiereinrichtung bewirkt. Nach Entnahme des Spülguts entfällt die Klemmkraft an den Klemmbacken und die Federschenkel können weiter nach innen nachfedern. Der sich dadurch verringerte Rastpunkt zwischen Federbügel und Kupplungseinrichtung wird mit Hilfe der Druckfeder überwunden. Durch die Entnahme des Spülguts löst sich mittels der Druckfeder somit die Verrastung der Rast- oder Arretiereinrichtung, die Kupplungseinrichtung wird zurück in die Ausgangsposition bewegt und die Klemmeinrichtung wird in die Nicht-Klemmstellung überführt.

Die Kupplungseinrichtung weist ein vorzugsweise plattenförmig ausgebildetes Kupplungsteil sowie eine davon getragene Spülgutdüse auf. Das Kupplungsteil dient der abstützenden Aufnahme eines zu reinigenden Spülguts. Zudem trägt das Kupplungsteil eine Spülgutdüse, die im bestimmungsgemäßen Verwendungsfall die strömungstechnische Verbindung zwischen dem von der Rohrleitung bereitgestellten Anschlussstutzen einerseits und dem Innenraum des zu reinigenden Spülguts andererseits ausbildet.

Für einen fluiddichten Anschluss des zu reinigenden Spülguts an die Spülgutdüse ist eine Dichtung vorgesehen, die spülgutseitig am Kupplungsteil angeordnet ist. Diese Dichtung verfügt bevorzugterweise über einen Dichtungsstutzen, der die Spülgutdüse ringartig umschließt. Die Dichtung stellt ferner eine Anlagefläche bereit, die ausgehend vom Dichtungsstutzen konisch verlaufend ausgebildet ist. Auf diese Anlagefläche liegt das zu reinigende Spülgut im bestimmungsgemäßen Verwendungsfall stirnseitig an. Die konische Anlagefläche weist in einem Bereich vorzugsweise eine Ablaufvertiefung auf, um zu verhindern, dass sich das Spülmedium am Kupplungsteil bis zur Höhe des Dichtungsstutzens ansammelt und dadurch das Spülgut dort schlechter umspült und/oder es zu einer Verschleppung des Spülmediums kommt.

Insgesamt wird somit eine Abdichtung zwischen dem zu reinigenden Spülgut und der Spülgutdüse einerseits sowie dem zu reinigenden Spülgut und dem Kupplungsteil andererseits erreicht. Dabei ist die vorbeschriebene Ausgestaltung der Dichtung größenunabhängig vom zu reinigenden Spülgut, womit die erfindungsgemäße Spülvorrichtung größenuniversal einsetzbar ist.

Gemäß einem weiteren Merkmal der Erfindung ist vorgesehen, dass das Kupplungsteil Durchtrittsöffnungen aufweist, durch die hindurch jeweils ein Federschenkel der als Klemmeinrichtung dienenden Bügelfeder geführt sind. Diese konstruktive Ausgestaltung erbringt eine Wirkverbindung zwischen dem Kupplungsteil der Kupplungseinrichtung einerseits und den Federschenkeln der Klemmeinrichtung andererseits. Infolge dieser Wirkverbindung kommt es bei einer Verfahrbewegung der Kupplungseinrichtung und damit auch des Kupplungsteils in Höhenrichtung zu einer Einfederbewegung der Federschenkel in Richtung auf das zu klemmende Spülgut bzw. zu einer Ausfederbewegung der Federschenkel in entgegengesetzter Richtung, je nach Verfahrbewegungsrichtung des Kupplungsteils. Dabei sind die Federschenkel jeweils abgewinkelt ausgebildet und stellen so eine Führungskontur für das Kupplungsteil bereit. Bevorzugt ist indes eine Ausgestaltung der Federschenkel, demgemäß ein Verfahren des Kupplungsteils aus der Ausgangsstellung in Höhenrichtung nach unten ein Einfedern der Federschenkel mit dem Ergebnis stattfindet, dass die von den Federschenkeln endseitig bereitgestellten Klemmbacken an dem zu reinigenden Spülgut zu liegen kommen und dieses zwischen sich einklemmen. In dieser Klemmstellung ist das Kupplungsteil relativ gegenüber den Federnschenkeln verrastet, zu welchem Zweck die Federschenkel bevorzugterweise über entsprechende Rastrippen verfügen, an denen das Kupplungsteil unter Druckfedervorspannung nach Art eines Anschlags anliegt. Nach einer manuellen Lösung dieser Rastverbindung verfährt das Kupplungsteil druckfederkraftbelastet in Höhenrichtung nach oben, infolgedessen es zu einem Ausweiten der Federschenkel nach außen, d. h. in einer Richtung weg vom Spülgut kommt, infolgedessen sich die Klemmbacken der Federschenkel vom Spülgut lösen und die Lagefixierung des Spülguts infolge der Klemmung aufgelöst wird. Die Rastverbindung löst sich insbesondere auch dann, wenn das in der Klemmeinrichtung fixierte Spülgut wie bereits beschrieben aus der Vorrichtung entnommen wird.

Die erfindungsgemäße Spülvorrichtung erbringt eine Reihe von Einzelvorteilen, die in Kombination darüber hinaus auch synergetische Effekte liefern. So wird das zu reinigende Spülgut in seiner lagefixierten Stellung lediglich durch die beiden Klemmbacken, d. h. durch nur zwei Kontaktstellen gehalten. Damit ist die Außenoberfläche des zu reinigenden Spülguts für eine optimierte Reinigung besser zugänglich. Der bisherige großflächige Spülschatten durch das das Spülgut umschließende Kupplungsteil entfällt. Ferner ist die Verwendung und die Notwendigkeit von nur einer erfindungsgemäßen Spülvorrichtung als universaler Anschlussadapter für alle bekannten Dentalinstrumente ein wesentlicher Vorteil. Sowohl Turbinen als auch Hand- und Winkelstücke lassen sich mit einer erfindungsgemäßen Spülvorrichtung adaptieren. Bisher waren für diese Funktion eine Mehrzahl unterschiedlicher Adapter bzw. Kupplungsteile notwendig.

Von Vorteil ist des Weiteren die erhöhte Aufnahmekapazität bei gleichem Raumangebot. Dies ist dadurch erreicht, dass die erfindungsgemäße Spülvorrichtung konstruktionsbedingt kleinerer Außenabmessungen als vorbekannte Spülvorrichtungen aufweist, so dass auch nebeneinander liegende Anschlüsse einer Rohrleitung gleichzeitig verwendet werden können. Dies war bislang nicht möglich.

Ferner ist die Handhabung vereinfacht. Das zu reinigende Spülgut lässt sich sowohl in einfacher Weise adaptieren als auch nach erfolgter Reinigung wieder entnehmen, ohne dass ein Austausch von Kupplungsteilen für in ihren Abmessungen unterschiedliche Spülgüter erforderlich ist.

Weitere Merkmale und Vorteile der Erfindung ergeben sich aus der nachfolgenden Beschreibung anhand der Figuren. Dabei zeigen:
- Figur 1a: in schematisch perspektivischer Darstellung eine Spülgutaufnahme in der Ausgestaltung eines Spülkorbs;
- Figur 1b: in schematisch perspektivischer Darstellung eine zur Figur 1a alternativen Spülgutaufnahme;
- Figur 2a: in schematisch perspektivischer Darstellung eine Detailansicht der Spülgutaufnahme nach Figur 1a;
- Figur 2b: in schematisch perspektivischer Darstellung eine Detailansicht der Spülgutaufnahme nach Figur 1b;
- Figur 3: in schematisch perspektivischer Darstellung eine erfindungsgemäße Vorrichtung;
- Figur 4: in schematisch perspektivischer Darstellung eine erfindungsgemäße Vorrichtung mit aufgenommenen Spülgut;
- Figur 5: in schematischer Seitenansicht eine erfindungsgemäße Vorrichtung und
- Figur 6: in schematischer Seitenansicht eine erfindungsgemäße Vorrichtung mit aufgenommenen Spülgut.

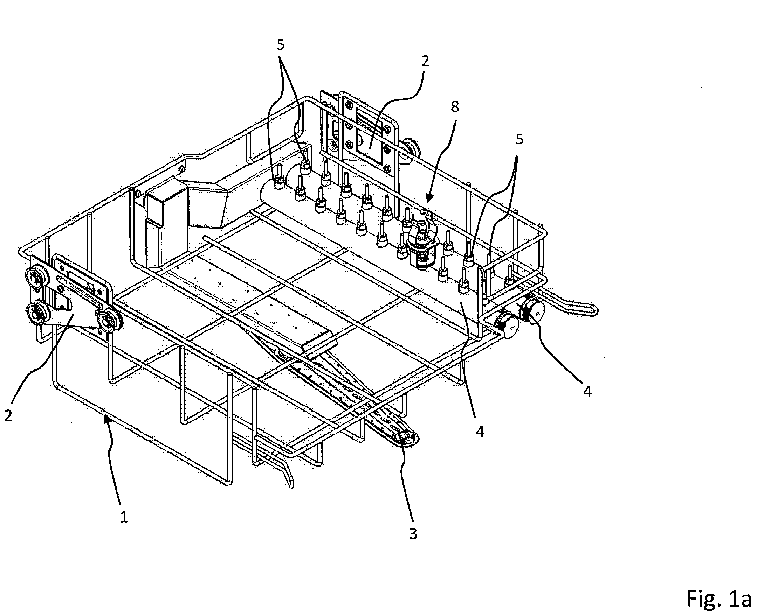

Figur 1a lässt in schematisch perspektivischer Darstellung eine Spülgutaufnahme 1 in der Ausgestaltung eines Spülkorbs erkennen. Im bestimmungsgemäßen Verwendungsfall ist die Spülgutaufnahme 1 verfahrbar vom Spülraum einer in den Figuren nicht näher dargestellten Geschirrspülmaschine, beispielsweise eines programmgesteuerten Reinigungs- und Desinfektionsautomaten aufgenommen. Die Spülgutaufnahme 1 verfügt zu diesem Zweck über Rollenaufnahmen 2, die im bestimmungsgemäßen Verwendungsfall von entsprechend ausgebildeten Widerlagern der in den Figuren nicht näher dargestellten Geschirrspülmaschine bereitgestellt sind.

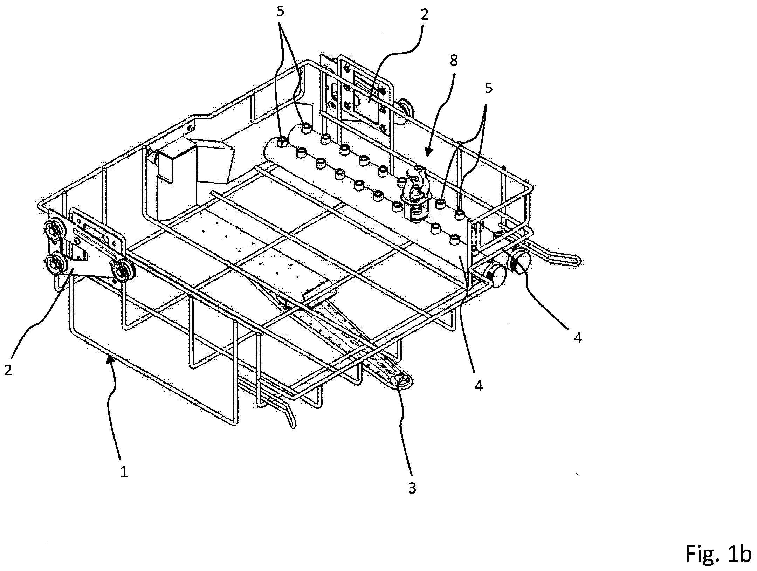

Im gezeigten Ausführungsbeispiel verfügt die Spülgutaufnahme 1 über einen daran verdrehbar angeordneten Sprüharm 3. Im bestimmungsgemäßen Verwendungsfall wird über diesen Sprüharm 3 Spülflotte in Richtung auf das von der Spülgutaufnahme 1 beherbergte und zu reinigende Spülgut abgegeben. Die über den Spülarm 3 abgegebene Spülflotte dient der Außenreinigung von in Figur 1 nicht näher dargestellten Spülgütern.

Zum Zwecke der Innenreinigung von Spülgütern stellt die Spülgutaufnahme 1 zwei Rohrleitungen 4 bereit, die jeweils eine Mehrzahl von Anschlusseinrichtungen 5 aufweisen. An diese Anschlusseinrichtungen 5 können jeweils zu reinigende Spülgüter angeschlossen werden, was im bestimmungsgemäßen Verwendungsfall zu einer Innenreinigung der Spülgüter führt. Die Rohrleitungen 4 mit ihren daran ausgebildeten Anschlusseinrichtungen 5 werden auch als Injektorleisten bezeichnet.

Für den Anschluss eines zu reinigenden Spülgutes an eine Injektorleiste dient je Anschlusseinrichtung 5 eine Vorrichtung 8 gemäß der Erfindung, auch Adapter oder Anschlussadapter genannt. Die erfindungsgemäße Vorrichtung 8 dient mithin dazu, einen strömungstechnischen Anschluss zwischen innen zu reinigendem Spülgut einerseits und zugehöriger Anschlusseinrichtung 5 andererseits auszubilden.

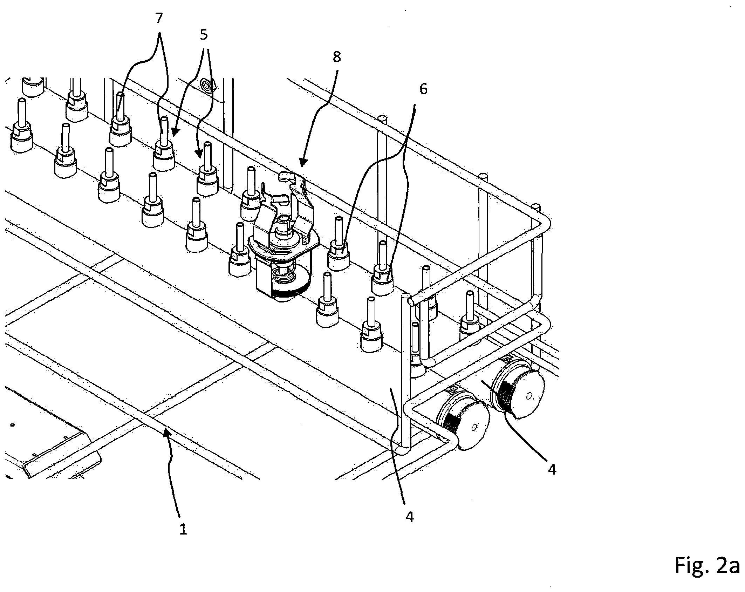

Figur 2a lässt in schematischer Darstellung in einer Detailansicht der Ausführung nach Figur 1a die Anschlusseinrichtungen 5 sowie exemplarisch eine mit einer Anschlusseinrichtung 5 gekoppelte erfindungsgemäße Vorrichtung 8 erkennen. Wie sich aus Figur 2a im Besonderen entnehmen lässt, stellt jede Anschlusseinrichtung 5 ein Anschlusselement 6 einerseits und einen Anschlussstutzen 7 andererseits bereit.

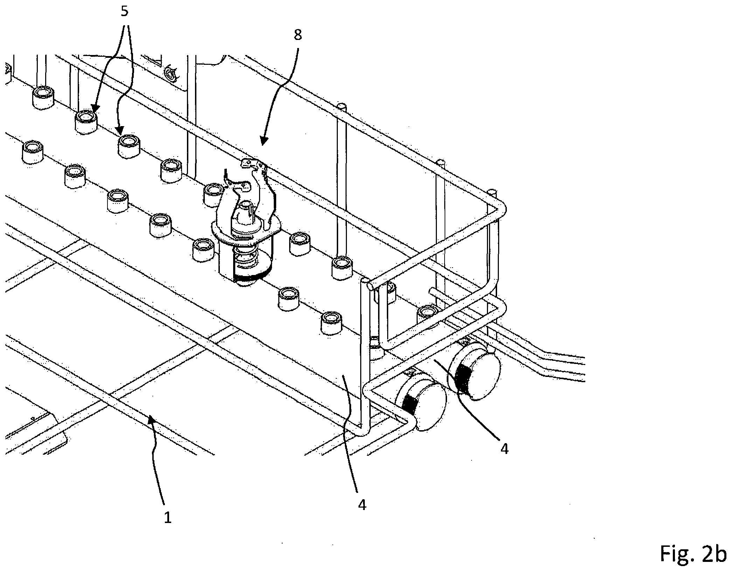

Eine zu den Figuren 1a und 2a alternative Ausführung zeigen die Figuren 1b und 2b. Diese unterscheiden sich von der Ausführung nach Figur 1a und 2a dadurch, dass das Anschlusselement 6 und der Anschlussstutzen 7, anders als in der Ausführung nach den Figuren 1a und 2a, nicht von der Anschlusseinrichtung 5 bereitgestellt werden, sondern integraler Bestandteil der Vorrichtung 8 sind.

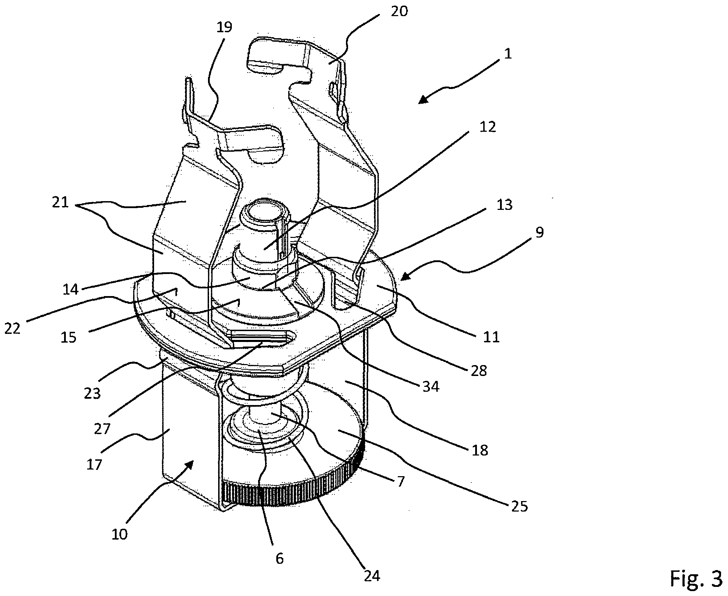

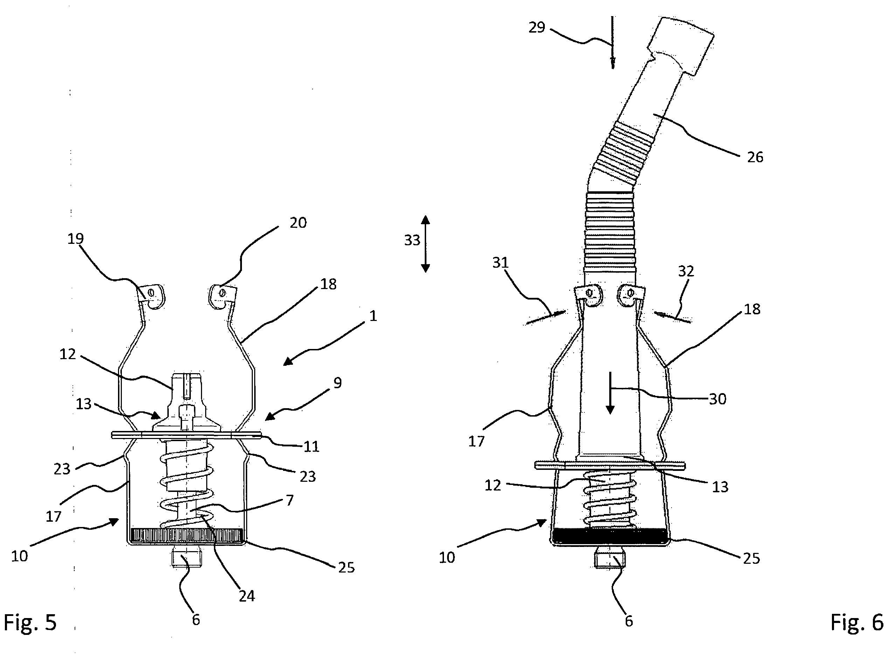

Figur 3 lässt in schematisch perspektivischer Darstellung eine zu dem Ausführungsbeispiel von Figur 1b und 2b korrespondierende erfindungsgemäße Vorrichtung 8 im Detail erkennen. Zum Zwecke der Anbindung der Vorrichtung 8 an die Anschlusseinrichtung 5 dient ein Halteelement 25, das im gezeigten Ausführungsbeispiel als werkzeuglos zu betätigende Gewindescheibe ausgebildet ist, wie insbesondere etwa Figur 5 entnehmbar. Das Halteelement 25 ist fest mit dem Anschlusselement 6 und dem Anschlussstutzen 7 verbunden. Das Anschlusselement 6 weist ein Außengewinde auf, das mit einem von der Anschlusseinrichtung 5 bereitgestellten Innengewinde zusammenwirkt. Mittels des Halteelements 25 kann die Vorrichtung 8 an die Anschlusseinrichtung 5 angeschlossenen werden, wobei das Anschlusselement 6 in das von der Anschlusseinrichtung 5 bereitgestellte Innengewinde hineingeschraubt wird. Bei der alternativen Ausführungsform gemäß den Figuren 1a und 1b, bei der das Anschlusselement 6 und der Anschlussstutzen 7 von der Anschlusseinrichtung 5 bereitgestellt werden, kann das Halteelement 25 eine Bohrung mit einem Inngewinde aufweisen, dass mit einem Außengewinde des Anschlusselements 6 zusammenwirkt. Zur Anbindung der erfindungsgemäßen Vorrichtung an die Anschlusseinrichtung 5 wird die Vorrichtung 8 auf den Anschlussstutzen 7 aufgesteckt und das Halteelement 25 auf das Anschlusselement 6 aufgeschraubt.

Die erfindungsgemäße Vorrichtung 8 verfügt des Weiteren über eine Kupplungseinrichtung 9. Diese Kupplungseinrichtung 9 stellt ein plattenförmiges Kupplungsteil 11 sowie eine davon getragene Spülgutdüse 12 bereit. Dabei stützt sich das Kupplungsteil 11 unter Zwischenordnung einer Druckfeder 24 relativ gegenüber dem Anschlusselement 6 ab, infolgedessen das plattenförmige Kupplungsteil 11 bestrebt ist, mit Bezug auf die Zeichnungsebene nach Figur 3 nach oben zu verfahren.

Die Kupplungseinrichtung 9 verfügt des Weiteren über eine Dichtung, die auf der dem Halteteil 25 abgewandten Seite des Kupplungsteils 11 angeordnet ist. Die Dichtung 13 verfügt dabei über einen Dichtungsstutzen 14, der die Spülgutdüse 12 ringartig umgibt, sowie über eine Anlagefläche 15, die vom Dichtungsstutzen 14 konisch verlaufend abgeschrägt ist. Die konische Anlagefläche 15 weist eine Ablaufvertiefung 34 auf. Wie die Darstellung nach Figur 3 erkennen lässt, greift der von der Anschlusseinrichtung 5 bereitgestellte oder mit der Anschlusseinrichtung 5 über das Anschlusselement verbindbare Anschlussstutzen 7 in die Spülgutdüse 12 ein, womit eine strömungstechnische Verbindung zwischen Spülgutdüse 12 einerseits und Anschlusseinrichtung 5 andererseits geschaffen ist.

Die erfindungsgemäße Vorrichtung verfügt des Weiteren über eine Klemmeinrichtung 10, die im gezeigten Ausführungsbeispiel als Bügelfeder ausgebildet ist. Es sind eine Federbasis 16 sowie zwei daran angeordnete Federschenkel 17 und 18 vorgesehen. Im endmontierten Zustand kommt die Federbasis 16 zwischen einer die Anschlusseinrichtung 5 bereitstellenden Rohrleitung 4 einerseits und dem Halteelement 25 andererseits zu liegen, so dass über das Halteteil 25 eine Lagefixierung der Klemmeinrichtung 10 relativ gegenüber der Rohrleitung 4 sichergestellt ist.

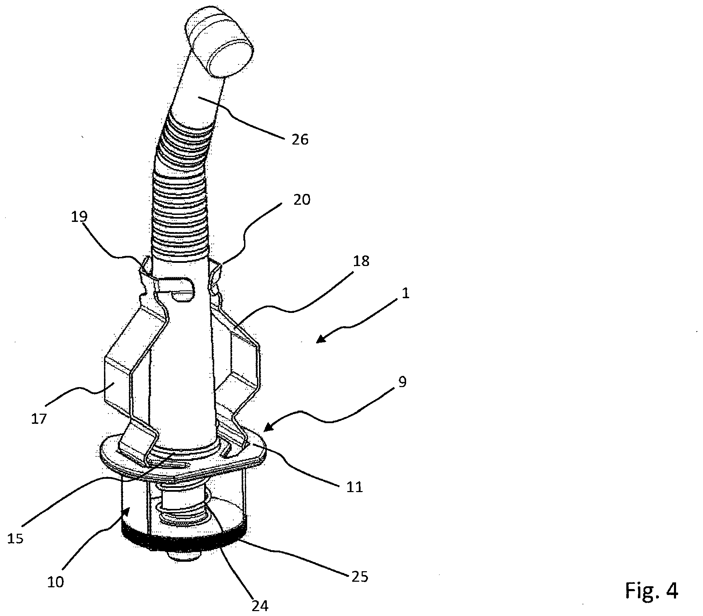

Jeder der beiden Federschenkel 17 und 18 verfügt jeweils endseitig über eine Klemmbacke 19 bzw. 20. Im Falle der Spülgutaufnahme durch die Vorrichtung 8 liegen diese Klemmbacken 19 und 20 am zu reinigenden Spülgut 26 an, wie dies Fig. 4 erkennen lässt.

Das Kupplungsteil 11 der Kupplungseinrichtung 9 verfügt über Öffnungen 27 bzw. 28. Durch diese Durchtrittsöffnung 27 und 28 hindurch sind die Federschenkel 17 bzw. 18 geführt, wobei der Federschenkel 17 der Durchtrittsöffnung 27 und der Federschenkel 18 der Durchtrittsöffnung 28 zugeordnet sind.

Die beiden Federschenkel 17 und 18 sind jeweils aus einer Mehrzahl einzelner Segmente 21 gebildet, die abgewinkelt zueinander angeordnet sind. Infolge dieser Ausgestaltung entsteht je Federschenkel 17 bzw. 18 eine Führungskontur 22, die mit der zugehörigen Durchtrittsöffnung 27 bzw. 28 zusammenwirkt. Ein jeder Federschenkel 17 bzw. 18 stellt des Weiteren jeweils einen Anschlag 23 in der Ausgestaltung eines rippenförmigen Vorsprungs bereit.

Figur 3 lässt die erfindungsgemäße Vorrichtung 8 in ihrer Ausgangslage erkennen, wohingegen Figur 4 die Vorrichtung 8 in bestücktem Zustand zeigt, d. h. die Vorrichtung 8 hat ein zu reinigendes Spülgut 26 bestimmungsgemäß aufgenommen, welches mittels der Klemmeinrichtung 10 in seiner relativen Lage zum Anschlussstutzen 7 fixiert ist.

Die bestimmungsgemäße Verwendung der erfindungsgemäßen Vorrichtung ergibt sich insbesondere aus einer Zusammenschau der Figuren 5 und 6.

Wie die Figuren 5 und 6 erkennen lassen, ist das Kupplungsteil 11 der Kupplungseinrichtung 9 in Höhenrichtung 33 relativ verfahrbar zum Anschlussstutzen 7 ausgebildet. Dabei kommt es infolge einer Verfahrbewegung der Kupplungseinrichtung 9, d. h. des Kupplungsteils 11 zu einer Betätigung der Klemmeinrichtung 10. Diese Betätigung der Klemmeinrichtung 10 ergibt sich aufgrund des Zusammenwirkens der Federschenkel 17 und 18 der Klemmeinrichtung 10 mit den vom Kupplungsteil 11 der Kupplungseinrichtung 9 bereitgestellten Durchtrittsöffnungen 27 und 28. Dabei sorgt die Führungskontur 22 der beiden Federschenkel 17 und 18 dafür, dass bei einer Verfahrbewegung des Kupplungsteils 11 mit Bezug auf die Zeichnungsebene nach Figur 5 nach unten ein Einschwenken der beiden Federschenkel 17 und 18, d. h. eine Einwärtsbewegung der Federschenkel 17 und 18 in Richtung auf ein von der Vorrichtung 8 aufgenommenes Spülgut 24 erfolgt. Dies machen die in Figur 6 eingezeichneten Pfeile 29, 30, 31 und 32 deutlich.

Infolge einer Verfahrbewegung des Kupplungsteils 11 mit Bezug auf die Zeichnungsebene nach Figur 5 nach unten wird die zwischen Anschlusselement 6 und Kupplungsteil 11 angeordnete Druckfeder 24 zusammengedrückt. Insoweit steht das Kupplungsteil 11 in der Stellung gemäß Figur 6, in der das zu reinigende Spülgut 26 durch Klemmung gehalten ist, unter Federvorspannung. In dieser Lage ist ein federinduziertes Verfahren des Kupplungsteils 11 mit Bezug auf die Zeichnungsebene nach Figur 5 bzw. 6 nach oben dadurch verhindert, dass das Kupplungsteil 11 an den von den Federschenkeln 17 und 18 jeweils bereitgestellten Anschlägen 23 anliegt, mithin eine Verrastung des Kupplungsteils 11 gegeben ist. Bedienerseitig kann diese Verrastung durch leichtes Einfedern der Federschenkel 17 und 18 aufgehoben werden, infolgedessen das Kupplungsteil 11 druckfederkraftbetrieben in Höhenrichtung 33 nach oben wandert, womit infolge der von den Federschenkeln 17 und 18 jeweils bereitgestellten Führungskontur 22 ein Ausfedern der Federschenkel 17 und 18 stattfindet, was alsdann eine Freigabe des Spülguts 24 zur Folge hat. Das Spülgut kann auch manuell nach oben entnommen werden. Dadurch wird das Ausfedern ermöglicht und das Kupplungsteil kehrt durch die Druckfeder in die Ausgangsposition zurück.

Mittels der Spülgutdüse 12 wird die strömungstechnische Verbindung zwischen dem zu reinigenden Spülgut 24 und dem Spülkreislauf, d. h. der zugehörigen Anschlusseinrichtung 5 hergestellt. Zudem wird die Spülgutdüse 12 mitels des Kupplungsteils 11 axial geführt, wobei sie in Kombination mit dem Anschlussstutzen 7 eine teleskopierbare Anschlussleitung ausbildet. Das Kupplungsteil 11 setzt eine Bewegung der Kupplungseinrichtung 9 in Höhenrichtung 33 in eine Verformung und damit im Falle einer Abwärtsbewegung in eine Haltekraft der Bügelfeder um. Mit der Dichtung 13 wird die Kontaktstelle zwischen Kupplungsteil 11 und zu reinigendem Spülgut 24 abgedichtet, so dass Druckverluste vermieden sind. Die Druckfeder 24 stellt die Rückstellkraft zur Verfügung, mit der die Ausgangsposition der erfindungsgemäßen Vorrichtung gemäß Figur 5 nach Entnahme eines gereinigten Spülguts 26 wieder hergestellt wird.

### Bezugszeichenliste

- 1: Spülgutaufnahme
- 2: Rollenaufnahme
- 3: Sprüharm
- 4: Rohrleitung
- 5: Anschlusseinrichtung
- 6: Anschlusselement
- 7: Anschlussstutzen
- 8: Vorrichtung (Adapter)
- 9: Kupplungseinrichtung
- 10: Klemmeinrichtung (Bügelfeder)
- 11: Kupplungsteil
- 12: Spülgutdüse
- 13: Dichtung
- 14: Dichtungsstutzen
- 15: Anlagefläche
- 16: Federbasis
- 17: Federschenkel
- 18: Federschenkel
- 19: Klemmbacke
- 20: Klemmbacke
- 21: Segment
- 22: Führungskontur
- 23: Anschlag
- 24: Druckfeder
- 25: Halteteil
- 26: Spülgut
- 27: Öffnung
- 28: Öffnung
- 29: Pfeil
- 30: Pfeil
- 31: Pfeil
- 32: Pfeil
- 33: Höhenrichtung
- 34: Ablaufvertiefung

## Patentansprüche

1. Vorrichtung zur Innendurchspülung von dentalem Spülgut, insbesondere Turbinen sowie Hand- und Winkelstücken, mit einer Kupplungseinrichtung (9) zum Anschluss des Spülguts (26) an einen mit einer Anschlusseinrichtung (5) einer Rohrleitung (4) verbundenen oder damit verbindbaren Anschlussstutzen (7) und mit einer Klemmeinrichtung (10) zur Lagefixierung eines an die Kupplungseinrichtung (9) angeschlossenen Spülguts (26),
**dadurch gekennzeichnet dass** die Kupplungseinrichtung (9) zur Betätigung der Klemmeinrichtung (10) relativ verfahrbar zum Anschlussstutzen (7) ausgebildet ist, wobei die Kupplungseinrichtung (9) ein Kupplungsteil (11) und eine davon getragene Spülgutdüse (12) aufweist,
und wobei das Kupplungsteil (11) Durchtrittsöffnungen (27, 28) aufweist, durch die hindurch jeweils ein Federschenkel (17, 18) einer als Klemmeinrichtung (10) dienenden Bügelfeder geführt sind.

2. Vorrichtung nach Anspruch 1,
**dadurch gekennzeichnet,**
**dass** die Kupplungseinrichtung (9) in Höhenrichtung (33) des Anschlussstutzens (7) relativ verfahrbar zum Anschlussstutzen (7) ausgebildet ist.

3. Vorrichtung nach Anspruch 1 oder 2,
**dadurch gekennzeichnet,**
**dass** die Klemmeinrichtung (10) eine Bügelfeder aufweist, deren Federschenkel (17, 18) endseitig jeweils eine mit dem Spülgut (26) zusammenwirkende Klemmbacke (19, 20) aufweisen.

4. Vorrichtung nach einem der vorhergehenden Ansprüche,
**dadurch gekennzeichnet,**
**dass** die Kupplungseinrichtung (9) gegenüber dem Anschlussstutzen (7) unter Zwischenschaltung einer Druckfeder (24) abgestützt ist.

5. Vorrichtung nach einem der vorhergehenden Ansprüche,
**dadurch gekennzeichnet,**
**dass** die Kupplungseinrichtung (9) ein plattenförmiges Kupplungsteil (11) aufweist.

6. Vorrichtung nach einem der vorhergehenden Ansprüche,
**dadurch gekennzeichnet,**
**dass** das Kupplungsteil (11) spülgutseitig eine Dichtung (13) aufweist.

7. Vorrichtung nach Anspruch 6,
**dadurch gekennzeichnet,**
**dass** die Dichtung (13) die Spülgutdüse (12) ringartig umschließt.

8. Vorrichtung nach Anspruch 6 oder 7,
**dadurch gekennzeichnet,**
**dass** die Dichtung (13) eine konisch verlaufende Anlagefläche (15) bereitstellt.

9. Vorrichtung nach einem der vorhergehenden Ansprüche,
**dadurch gekennzeichnet,**
**dass** die Federschenkel (17, 18) abgewinkelt ausgebildet sind und jeweils eine Führungskontur (22) für das Kupplungsteil (11) bereitstellen.

## Claims

1. Device for internally rinsing dental items to be rinsed, in particular turbines and hand pieces and angle pieces, comprising a coupling means (9) for connecting the items (26) to be rinsed to a connecting piece (7) which is connected or can be connected to a connection means (5) of a pipe (4), and comprising a clamping means (10) for fixing the position of an item (26) to be rinsed that is connected to the coupling means (9),
**characterised in that** the coupling means (9) is designed to be movable relative to the connecting piece (7) in order to actuate the clamping means (10), the coupling means (9) comprising a coupling part (11) and a nozzle (12), supported thereby, for the items to be rinsed, and the coupling part (11) comprising through-openings (27, 28), through each of which a spring leg (17, 18) of a retaining spring acting as a clamping means (10) is guided.

2. Device according to claim 1,
**characterised in that**
the coupling means (9) can be moved relative to the connecting piece (7) in the vertical direction (33) of the connecting piece (7).

3. Device according to either claim 1 or claim 2,
**characterised in that**
the clamping means (10) comprises a retaining spring, the spring legs (17, 18) of which each comprise, at the end thereof, a clamping jaw (19, 20) that interacts with the item (26) to be rinsed.

4. Device according to any of the preceding claims,
**characterised in that**
the coupling means (9) is supported relative to the connecting piece (7) by the insertion of a compression spring (24).

5. Device according to any of the preceding claims,
**characterised in that**
the coupling means (9) comprises a planar coupling part (11).

6. Device according to any of the preceding claims,
**characterised in that**
the coupling part (11) comprises a seal (13) on the side for the item to be rinsed.

7. Device according to claim 6,
**characterised in that**
the seal (13) annularly surrounds the nozzle (12) for the item to be rinsed.

8. Device according to either claim 6 or claim 7,
**characterised in that**
the seal (13) provides a conical contact face (15).

9. Device according to any of the preceding claims,
**characterised in that**
the spring legs (17, 18) are angled and each provide a guide contour (22) for the coupling part (11).

## Revendications

1. Dispositif destiné au rinçage interne d'articles à rincer dentaires, en particulier turbines ainsi que pièces à main et angulaires, avec des moyens d'accouplement (9) destinés à la jonction de l'article à rincer (26) avec une tubulure de jonction (7) raccordée à des moyens de jonction (5) d'une conduite tubulaire (4) ou pouvant y être raccordée, et avec des moyens de serrage (10) pour la fixation en position d'un article à rincer (26) joint aux moyens d'accouplement (9),
**caractérisé en ce que** les moyens d'accouplement (9) sont, pour l'actionnement des moyens de serrage (10), constitués de façon déplaçable par rapport à la tubulure de jonction (7),
dans lequel les moyens d'accouplement (9) présentent une partie d'accouplement (11) et une buse d'article à rincer (12) portée par celle-ci,
et dans lequel la partie d'accouplement (11) présente des ouvertures de passage (27, 28) à travers lesquelles respectivement une branche de ressort (17, 18) d'un ressort en étrier servant de moyens de serrage (10) est guidée.

2. Dispositif selon la revendication 1,
**caractérisé en ce que**
les moyens d'accouplement (9) sont constitués de façon déplaçable par rapport à la tubulure de jonction (7) dans la direction en hauteur (33) de la tubulure de jonction (7).

3. Dispositif selon la revendication 1 ou 2,
**caractérisé en ce que**
les moyens de serrage (10) présentent un ressort en étrier dont les branches de ressort (17, 18) présentent, côté extrémité, respectivement une mâchoire de serrage (19, 20) coopérant avec l'article à rincer (26).

4. Dispositif selon l'une des revendications précédentes,
**caractérisé en ce que**
les moyens d'accouplement (9) sont, par rapport à la tubulure de jonction (7), soutenus avec interposition d'un ressort de pression (24).

5. Dispositif selon l'une des revendications précédentes,
**caractérisé en ce que**
les moyens d'accouplement (9) présentent une partie d'accouplement (11) en forme de plaque.

6. Dispositif selon l'une des revendications précédentes,
**caractérisé en ce que**
la partie d'accouplement (11) présente un joint d'étanchéité (13) côté article à rincer.

7. Dispositif selon la revendication 6,
**caractérisé en ce que**
le joint d'étanchéité (13) entoure la buse d'article à rincer (12) de façon annulaire.

8. Dispositif selon la revendication 6 ou 7
**caractérisé en ce que**
le joint d'étanchéité (13) fournit une surface d'appui (15) conique.

9. Dispositif selon l'une des revendications précédentes,
**caractérisé en ce que**
les branches de ressort (17, 18) sont constituées de façon coudée et fournissent respectivement un contour de guidage (22) pour la partie d'accouplement (11).
